# EUROPEAN PATENT SPECIFICATION

(11) **EP 0 687 691 B1**
(45) Date of publication and mention of the grant of the patent: **07.10.1998**
(21) Application number: 95112933.7
(22) Date of filing: 27.08.1991
(51) Int. Cl.: C08F 8/00, C08G 81/02, C07K 1/04

(54) **Resin for solid-phase peptide synthesis**
Harze für die Feststoff-Peptidsynthese
Résines pour la synthèse de peptides en phase solide

(30) Priority: 31.08.1990 US 576314; 14.06.1991 US 715289
(43) Date of publication of application: 20.12.1995
(62) Divisional of application: 91916082.0
(73) Proprietor: REGENTS OF THE UNIVERSITY OF MINNESOTA, Minneapolis, Minnesota 55415-1226 (US)
(72) Inventor: Barany, George, Falcon Heights, MN 55113 (US); Chang, Jane, Vernon Hills, IL 60061 (US); Sole, Nuria, Boston, Massachusetts 02108-1017 (US); Albericio, Fernando, E-08028 Barcelona (ES); Zalipsky, Samuel, c/o Sequus Pharmaceuticals, Menlo Park, California 94025 (US)
(74) Representative: Harvey, David Gareth

(56) References cited:
- EP-A- 0 187 391
- EP-A- 0 347 145
- CH-A- 602 822
- DE-A- 2 649 848
- US-A- 4 831 084

## Description

### Background of the Invention

Spacer arms are essential in many areas of modern biochemistry. Spacer arms can be defined as molecules which link one molecule to another molecule or to an inert support. Polyethylene glycol, for example, has been used advantageously to link enzymes to insoluble carriers and other biomolecules while retaining the activity of the enzyme. M. Stark and K. Holmberg, Biotech. and Bioeng., 34:942-950 (1989). This concept has important consequences for industrial processes using immobilized enzymes (e.g., affinity column purification processes), and for diagnostic assays (e.g., ELISA, (Enzyme linked immuno sorbant assay), assays). Two other areas in which polyethylene glycol spacer arms have been used are peptide synthesis and sequencing. The coupling rate of protected nucleotide and amino acid residues to inert supports, such as silica, membrane, and polystyrene supports, often increases commensurately with the separation of the reaction site from the support backbone. Similar effects have been shown for sequencing of solid-phase immobilized samples. J.K. Inman et al., In Solid Phase Methods in Protein Sequence Analysis, Previero and Coletti-Previero, (Eds.), Elsevier, North-Holland Biomed. Press, pp. 81-94 (1977).

The effectiveness of solid-phase nucleic acid or peptide synthesis or sequence analysis is affected by the solid-phase or support which anchors the reactive sites. Polystyrene gels or porous glass have both been utilized as solid supports for peptide sequencing, for example. In many applications, the solvents used in the process can cause the polystyrene particles to change in volume, which causes blocking of the reaction column and back pressure. Conversely, porous glass is completely rigid and does not change in volume, but the chemical properties of porous glass derivatives have lacked reproducibility. Polymer particles, such as polystyrene particles, which have been derivatized so that reactive groups can be attached to them, have proved useful in many applications. Polyethylene glycol (PEG) structures have been used as chemically inert spacer arms be because they are compatible with a wide range of solvents. Inman et al., ibid. The use of PEG spacer arms minimizes the steric effects caused by the support. PEG spacer arms provide another useful function in modifying the character of the pore space so that the support-bound reactive moiety is compatible with a wider range of solvents and reagents.

PEG-modified polystyrene (PEG-PS) resins have been described for use in solid-phase peptide sequencing. Inman et al., ibid. PEG-PS resins have also been utilized as phase transfer catalysts. W.M. McKenzie et al., J. Chem. Soc. Chem. Commun., p. 541-543 (1978); S.L. Regen et al., J. Amer. Chem. Soc., 101:116-120 (1979); J.G. Heffernan et al., J. Chem. Soc. Perkin II, p. 514-517 (1981); Y. Kimura et al., J. Org. Chem., 48:385-386 (1983); M. Tomoi et al., Reactive Polymers, 10:27-36 (1989). PEG-PS resins have been described as supports for solid-phase peptide synthesis. Becker et al., Makromol. Chem. Rapid Commun., 3:217-223 (1982); H. Hellermann, et al., Makromol. Chem., 184:2603-2617 (1983). However, PEG-PS resins prepared by the referenced methods suffer from several drawbacks. The reactions proceeded poorly with high molecular weight PEG (e.g., greater than 400 daltons) and symmetrical bifunctional PEG tended to form crosslinks. These problems were reduced by the anionic polymerization of ethylene oxide directly onto crosslinked polystyrene. Bayer et al., In Peptides: Structure and Function, V.J. Hruby and D.H. Rich (eds.), Proc. 8th Am. Peptide Symp. pp. 87-90, Pierce Chem. Co., Rockford, IL (1983). Bayer and Rapp, German Patent DE 3500180 A1 (1986). However, the PEG chain lengths are difficult to control using this method, and the uniformity of the PEG polymers is uncertain. Another problem with this process is that the polystyrene is functionalized using chloromethyl ether, which is highly toxic, and residual chloromethyl groups can cause side reactions during peptide synthesis.

Another method of making PEG graft copolymers is described by Zalipsky et al., In Peptides:Structure and Function, C.M. Deber, V.J. Hruby and K.D. Kopple (eds.), Proc. 9th Am. Pep. Symp., pp. 257-260, Pierce Chem. Co., Rockford, IL (1985). In this method, certain heterobifunctional PEG derivatives of defined molecular weight (i.e., 2000 to 4000 daltons) were used. However, these derivatives are not readily available, which hinders their commercialization.

A method of preparing non-toxic and efficient solid supports which can be used with a wide range of solvents would be valuable for use in solid-phase synthesis or sequencing of peptides or nucleic acids, or for other solid-phase applications.

### Summary of the Invention

The present invention pertains to polyethylene glycol derivatized graft supports.

This specification discloses methods for making the supports, and to methods of using the supports to synthesize peptides by solid-phase synthesis techniques. The PEG-graft supports of this invention comprise functionalized PEG derivatives which are covalently attached to solid supports.

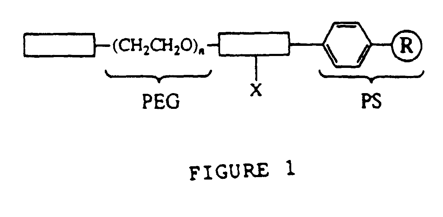

This invention provides a resin, for solid-phase peptide synthesis, constructed such that peptide synthesis can occur at a point other than the terminus of PEG. Such resin is illustrated in Figure 1 and further represented by the general Formula I: 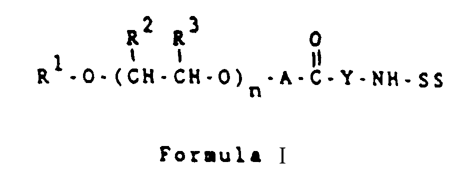 wherein Y is a diamino monocarboxylic acid residue that can optionally be protected by a N^{ω} protecting group; n is an integer from 5 to 150; SS is a solid support; A is a straight chain or branched C1-C10 alkyl group, such as methylene, ethylene, propylene, isopropylene, butylene and isobutylene; and R¹, R² and R³ are independently selected from the group consisting of hydrogen, alkyl groups and aryl groups.

The present invention provides several compact, commercially viable routes for making functionalized inert supports for solid-phase applications using monofunctional or homobifunctional polyethylene glycol as the starting material. The present PEC graft supports provide advantageous physical and mechanical properties for solid-phase peptide synthesis, nucleic acid synthesis, and other applications where immobilized molecules are used.

### Brief Description of the Figures

Figure 1 shows the general structure of a polyethylene glycol-polystyrene (PEG-PS) graft support; X illustrates the point from which biopolymer chain growth begins, and

Figure 4 is a schematic showing a series of reactions leading to a PEG-PS graft support in which the biopolymer can be synthesized from the Orn residue.

### Detailed Description of the Invention

The present invention pertains to resins comprising a functionalized polyethylene glycol derivative covalently linked to a solid support. As disclosed and claimed in patent EP A 91 91 6082.0, one such graft support comprises a symmetrical poly(oxyethylene) diamine derivative linked to the support and is represented by the following Formula : 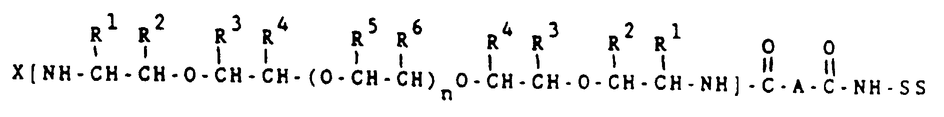 wherein n is an integer from 5 to 150; R¹, R², R³, R⁴, R⁵ and R⁶ are independently selected from the group consisting of hydrogen (H), and simple alkyl or aryl groups such as methyl, ethyl or phenyl; SS is a solid support; X is H or H₂N-B-NH-C(O)-A-C(O)-; and A and B are independently a straight chain or branched alkyl group, such as ethylene, propylene, isopropylene, butylene, isobutylene or the like up to C-10 in length (e.g., A derived from succinic, glutaric, adipic or other such acids; B derived from ethylenediamine or other aliphatic diamines); a CH-CH group (e.g., A derived from maleic acid) or an aromatic group (e.g., A derived from phthalic acid; B derived from phenylenediamine). The core portion of the formula, within the bracketed portion, corresponds to a series of polyoxyethylene diamine polymers. The amino group(s) can optionally be protected by known N^{ω} protecting groups.

One method for producing the graft supports of the above Formula is by reacting amino-functionalized core polymers with dicarboxylic acid derivatives, including anhydrides, to produce carboxyl-functional molecules. According to this method, the diamine polymer is reacted with at least two equivalents of the activated dicarboxylic acid derivative. Dicarboxylic acids which are useful in this method include alkyl diacids having up to about 12 carbon atoms, such as, for example, maleic, succinic, glutaric or adipic acid; anhydrides such as maleic, succinic or glutaric anhydride; or aromatic anhydrides, such as phthalic anhydride. In one embodiment of this method, the diamine polymer is reacted with succinic, maleic or glutaric anhydride to make representatives of the claimed compounds, bis(succinylated), bis(maleylated) or bis(glutarylated) PEG.

The PEG graft supports of EP A-91 91 6082.0 make it possible to assemble biopolymers at the PEG terminus, as shown in Figure 1 of that application. In the present invention, however, the resins are constructed such that peptide synthesis can occur at a point other than the PEG terminus. Such resins are illustrated in Figure 1 hereof and are further represented by the general Formula I : 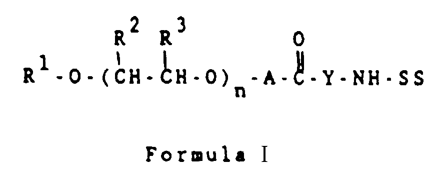 wherein Y is a diamino monocarboxylic acid residue that can optionally be protected by a N^{ω} protecting group; n is an integer from 5 to 150; SS is a solid support; A is a straight chain or branched C1-C10 alkyl group, such as methylene, ethylene, propylene, isopropylene, butylene and isobutylene; and R¹, R² and R³ are independently selected from the group consisting of hydrogen, alkyl groups and aryl groups.

A PEG graft support of this type (formula I) is synthesized using a monofunctionalized polyethylene glycol (e.g., PEG monoamine or PEG monomethyl ether) which has been derivatized. Commercially available PEG starting materials are either monofunctional Jeffamine ^{®} or PEG monomethyl ether which has methoxy and hydroxyl end groups. An amino group on PEG can be reacted with a dicarboxylic acid or anhydride as already described above in relation to the resin the subject of EPA 91 91 6082.0. Alternatively, carboxyl-functionalized derivatives of MPEG can be made by reaction with ethyl bromoacetate, 4-bromovalerate or isocyanacetate, followed by saponification, for example as shown in the equation below.

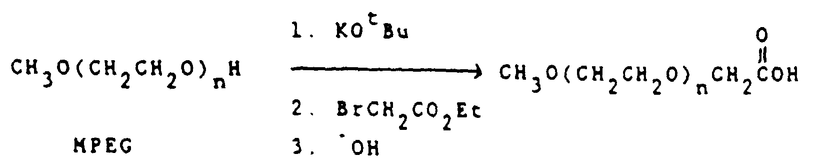

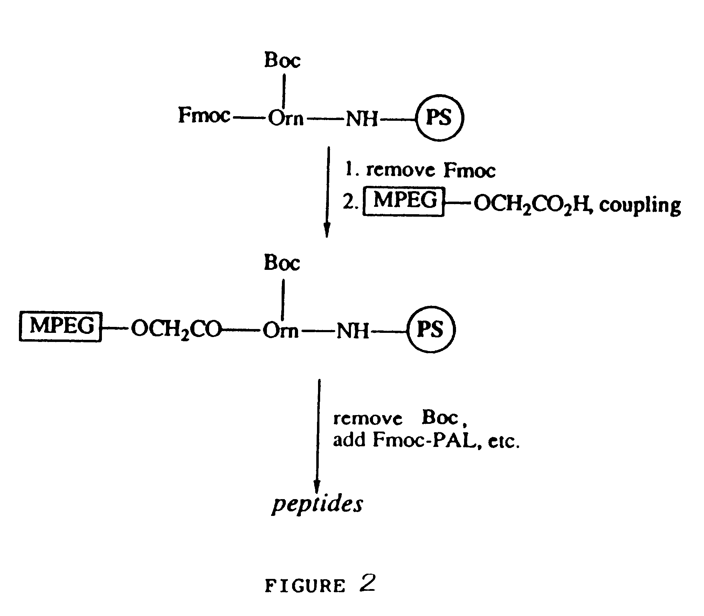

Figure 2 schematically illustrates the synthesis of a PEG resin of Formula I. According to this method, an N^{α}-Fmoc, (N^{α}-9-fluorenylmethyloxycarbonyl), N^{δ}-Boc-Orn-PS (N^{δ}-tert-butyloxycarbonyl-ornithine-PS resin (or a permutation on this theme) is deblocked selectively, and a monofunctional PEG-acid is coupled on to form a branch orthogonal to the ultimate direction of biopolymer chain growth. An ornithine residue (as shown in Figure 2) is used to link the PEG derivative to the solid support; however, other chemical moieties which have a carboxyl group and two amino groups can be used.

Polyethylene glycol-polystyrene (PEG-PS) graft supports made by the methods disclosed herein are particularly useful. PEG-PS supports made using the present PEG derivatives have several desirable characteristics for solid-phase applications: they swell in a variety of solvents, are stable under the conditior used in most solid-phase synthesis, and behave well in both batch and column reactors used in solid-phase applications, in particular, solid-phase peptide synthesis.

Solid-phase peptide synthesis typically begins with covalent attachment of the carboxyl end of a first amino acid to the solid support. The carboxyl group of an N^{α}-protected amino acid is covalently linked to a handle moiety which is attached to a point other that the PEG terminus (Figures 1 and 2). A "handle" is defined as a bifunctional spacer which serves to attach the initial amino acid residue to the polymeric support. One end of the handle incorporates a smoothly cleavable protecting group and the other end of the handle couples to the functionalized solid support. Handles which can be used with the present spacer arms in solid-phase peptide synthesis include, for example acid-labile p-alkoxybenzyl (PAB) handles, photolabile o-nitrobenzyl ester handles, and handles such as those described by Albericio et al., J. Org. Chem., 55:3730-3743 (1990) and references cited therein. The appropriate handles are coupled quantitatively in a single step onto the amino-functionalized supports to provide a general starting point of well-defined structures for peptide chain assembly. The handle protecting group is removed and the C-terminal residue of the N^{α}-protected first amino acid is coupled quantitatively to the handle. Once the handle is coupled to the solid-phase and the initial amino acid or peptide is attached to the handle, the general synthesis cycle proceeds. The synthesis cycle generally consists of deprotection of the N^{α}-amino group of the amino acid or peptide on the resin, washing, and, if necessary, a neutralization step, followed by reaction with a carboxyl-activated form of the next N^{α}-protected amino acid. The cycle is repeated to form the peptide or protein of interest. Solid-phase peptide synthesis methods using functionalized insoluble supports are well known. Merrifield, J. Am. Chem. Soc., 85:2149 (1963); Barany and Merrifield, In Peptides, Vol. 2, pp. 1-284 (1979); Barany et al., Int. J. Peptide Protein Res., 30:705-739 (1987).

The present PEG-derivatives are particularly useful as spacer arms, which separate the inert support from the reacting amino acids which are forming the peptide chain during the synthesis process. The choice of a spacer arm, which provides this distance, is a critical parameter in solid-phase applications.

In a preferred embodiment of the present invention, a variety of PEG spacer arms having an average molecular weight of about 2000 daltons were incorporated between amino functional groups on a polystyrene backbone and the point for attachment of the appropriate handles. The resultant PEG-PS graft supports contained approximately equal weight amounts PEG and PS. These supports showed reproducible advantage over PS supports with regard to physical and chemical properties such as swelling and in synthesis of model peptides. The PEG-PS supports of this invention allow peptide synthesis to be carried out using acetonitrile as the solvent for all reaction steps and washes. The control experiments using PS and employing acetonitrile as the solvent resulted in no peptide products.

In another embodiment, comparative experiments were carried out in which the difficult acyl carrier protein 65-74 decapeptide sequence was synthesized using Fmoc-amino acids. The peptide was produced in higher purity on this new PEG-PS than on either PS, Tentagel™ (a trademark of Rapp Chem. Tubingen, Germany) , or Pepsyn K™ (a trademark of Cambridge Research Biochem., Cambridge, England). The PEG-PS material proved highly suitable both for flow-through synthesis and batch operation. Negligible back-pressures were found even at high flow rates.

The general usefulness of the PEG-PS graft support was demonstrated by syntheses of a number of large, (e.g., having about 30 to 60 residues) complex peptide sequences, such as cecropin analogues, calcitonin, β-endorphin, corticotropin releasing factor, two zinc finger binding sequences, and several partial sequences of HIV-1 tat protein. The improvements in synthetic efficiency which resulted from use of the present PEG-PS linkers appear to originate from one or more of the following: (i) a spacer arm effect removing the reaction sites from the vicinity of the polymer backbone; (ii) a general environmental effect which modifies the hydrophobic nature of the resin with a concomitantly favorable influence on reaction rates; and (iii) a specific effect on conformationally difficult couplings due to decreased secondary structure (hydrogen bond formation).

The PEG derivatives can also be used in nucleic acid synthesis or sequencing as spacer arms or linkers between an inert support and the reacting nucleotide or nucleic acid. For example, the derivatives can be attached to polystyrene resins as described above and used in amidite-mediated DNA synthesis. The PEG-PS resin swells in acetonitrile, which is used as a solvent in the amidite coupling method.

The invention will now be further illustrated by the following examples:

### EXAMPLES

### Example 1. Preparation of a Carboxymethyl Derivative of Polyethylene Glycol Methyl Ether (MPEG)

A solution of polyethylene glycol methyl ether (MPEG), M_{W} 2000 (100 g, 50 mmol) in toluene (500 mL) was refluxed overnight for azeotropic drying, with water removed by a Dean-Stark trap attachment. The solution was cooled to 80°C, treated with potassium tert-butoxide (11.2 g, 0.1 mol), and then a mixture of ethyl bromoacetate (11 mL, 0.1 mol) and toluene (10 mL) was added over 30 minutes. Reaction continued for 8 hours at 90°C, following which the mixture was concentrated in a rotary evaporator to form a brown viscous melt. Dichloromethane (500 mL) was added to extract the polymer, and activated alumina (200 g) was added. After 30 minutes, the organic phase was collected upon filtration, partially concentrated (to - 100 mL), combined with ethyl ether (700 mL), and brought to -20°C for several hours. The precipitated polymer was collected by filtration, air-dried, and taken up in aqueous sodium hydroxide (1 N, 500 mL) for 3 hours, 25°C. This saponification reaction was quenched by acidification to pH 3 with concentrated aqueous HCl, and the product was extracted into dichloromethane (3 x 250 mL). The organic phase was dried (MgSO₄), partially concentrated (to - 100 mL), combined with ethyl ether (700 mL), and brought to -20°C for several hours. The final product was collected by filtration, and dried in vacuo over P₂O₅ for several days. Yield: 75 g (73%), titration of carboxyl groups 94% of theory. NMR data and elemental analysis in accord with structure.

### Example 2. Preparation of PE-Orn(Boc)-PS Resin

An aminomethyl polystyrene resin support (6.0 g, 0.95 mmol/g, 5.7 mmol) was derivatized with N^{α}-Fmoc, N^{δ}-Boc-ornithine by standard procedures. The resin was swollen and washed with dichloromethane, neutralized with 5% DIEA in dichloromethane (1 x 5 min + 3 x 10 min), and washed with dichloromethane. Coupling of the Orn derivative was mediated by N,N'-diisopropylcarbodiimide (DIPCDI)/HOBt in DMF method (3 equiv. each, 15 min preactivation, 40 mL reaction volume), 3 hours. Washing followed with DMF and dichloromethane. A ninhydrin test on a sample was nearly negative, but unreacted sites were blocked by capping with acetic anhydride (4.5 mL, 5 equiv.) and triethylamine (7.0 mL, 5 equiv.) in DMF (20 mL), 30 minutes. The protected Orn-PS resin (0.78 mmol/g, matches theoretical loading as adjusted for expected weight gain) was washed with DMF, dichloromethane, and treated with piperidine-DMF (1:4, v/v) (2 + 10 min) to remove selectively the N^{α}-Fmoc group. Further washings with DMF, dichloromethane, 2-propanol, and DMF-dichloromethane (3:7, v/v) prepared the resin for coupling of the MPEG-acid derivative, produced as described in Example 1. A solution of the MPEG-acid (37 g, 18 mmol) in DMF-dichloromethane (3:7 v/v, 75 mL) was combined with HOBt (2.75 g, 18 mmol) and DIPCDI (2.83 mL, 18 mmol) for 15 minutes, and was then added to the H-Orn(Boc)-PS resin. Coupling proceeded for 5 hours, giving a slightly positive ninhydrin test. A second coupling with smaller amounts of MPEG-acid derivative and activating agents (9 mmol scale), and acetylation by already-described procedures followed. The final MPEG-Orn(Boc)-PS resin was washed with dichloromethane, DMF, dichloromethane and methanol, and dried in vacuo over P₂O₅ for 48 hours. There was obtained 16.9 g of resin (excellent agreement with theoretical weight gain) with a loading of 0.31 mmol/g, comprising PEG:PS:Orn - 0.57:0.36:0.07 by weight (based on elemental analysis and amino acid analysis). Similar results were obtained when BOP/HOBt/NMM in DMF protocols were used for activation and coupling.

### Example 3. Continuous Flow Peptide Synthesis with Polyethylene Glycol-Polystyrene Graft (PEG-PS)

PEG-Orn(Boc)-PS prepared as described in Example 6 was treated with TFA-dichloromethane (1:1) (5 + 25 min) to remove selectively the N^{δ}-Boc group. There followed washing with dichloromethane, neutralization with 5% DIEA in dichloromethane and dichloromethane. An Fmoc-PAL handle was added, and a xilliGen/Biosearch model 9050 peptide synthesizer was used with a DIPCDI + HOBt coupling protocol (0.05 M concentrations), to make the challenging deca-alanyl-valine sequence. In a consecutive run, a normal polystyrene support was used to make the same sequence. The purity of the peptide based on HPLC was 77% when PEG-PS was used, as compared to 53% when PS was used.

## Claims (Claims for the following Contracting State(s): BE, CH, LI, DE, DK, FR, GB, IT, NL, SE)

1. A resin for solid-phase peptide synthesis represented by the general formula: 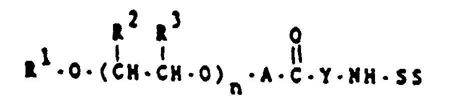 wherein Y is a diamino monocarboxylic acid residue that can optionally be protected by a N^{ω} protecting group; n is an integer from 5 to 150; SS is a solid support; A is a straight chain or branched C1-C10 alkyl group; and R¹, R² and R³ are independently selected from the group consisting of hydrogen, alkyl groups and aryl groups.

2. A resin of claim 1, wherein the solid support is selected from the group consisting of amino-functional membranes, porous glass, silica, polystyrenes, polydimethylacrylamides, cotton and paper.

3. A resin of claim 2, wherein the polystyrene is selected from the group consisting of amino-polystyrene, aminomethyl polystyrene, aminoacyl polystyrene and p-methylbenzhydrylamine polystyrene.

## Claims (Claims for the following Contracting State(s): ES)

1. A method for making a support for solid-phase peptide synthesis comprising making the support from a resin represented by the general formula: 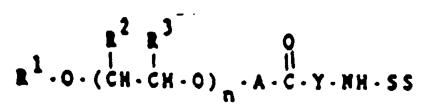 wherein Y is a diamino monocarboxylic acid residue that can optionally be protected by a N^{ω} protecting group; n is an integer from 5 to 150; SS is a solid support; A is a straight chain or branched C1-C10 alkyl group; and R¹, R² and R³ are independently selected from the group consisting of hydrogen, alkyl groups and aryl groups.

2. A method of claim 1, wherein the solid support is selected from the group consisting of amino-functional membranes, porous glass, silica, polystyrenes, polydimethylacrylamides, cotton and paper.

3. A method of claim 2, wherein the polystyrene is selected from the group consisting of amino-polystyrene, aminomethyl polystyrene, aminoacyl polystyrene and p-methylbenzhydrylamine polystyrene.

## Patentansprüche (Patentansprüche für folgende(n) Vertragsstaat(en): BE, CH, LI, DE, DK, FR, GB, IT, NL, SE)

1. Harz für die Festphasen-Peptid-Synthese der allgemeinen Formel: 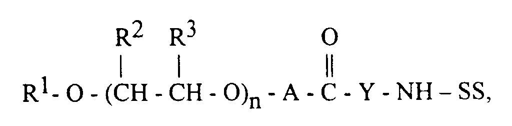 worin Y ein Diaminomonocarbonsäure-Rest ist, der wahlweise geschützt werden kann durch eine N^{ω} -Schutzgruppe; n eine ganze Zahl von 5 bis 150 ist; SS ein fester Träger ist; A eine geradkettige oder verzweigte C1- bis C10- Alkyl-Gruppe ist; und Rl, R2 und R3 unabhängig von einander ausgewählt sind aus der Gruppe bestehend aus Wasserstoff, Alkyl-Gruppen und Aryl-Gruppen.

2. Harz nach Anspruch 1, worin der feste Träger ausgewählt ist aus der Gruppe bestehend aus aminofunktionalen Membranen, porösem Glas, Siliciumdioxid, Polystyrolen, Polydimethylacrylamiden, Baumwolle und Papier.

3. Harz nach Anspruch 2, worin das Polystyrol ausgewählt ist aus der Gruppe bestehend aus Aminopolystyrol, Aminomethylpolystyrol, Aminoacylpolystyrol und p-Methylbenzhydrylaminpolystyrol.

## Patentansprüche (Patentansprüche für folgende(n) Vertragsstaat(en): ES)

1. Verfahren zur Herstellung eines Trägers für die Festphasen-Peptid-Synthese, indem der Träger aus einem Harz der allgemeinen Formel hergestellt wird: 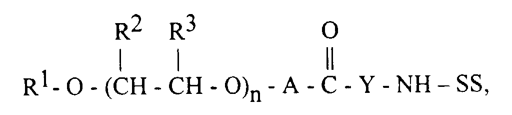 worin Y ein Diaminomonocarbonsäure-Rest ist, der wahlweise geschützt werden kann durch eine N^{ω} -Schutzgruppe; n eine ganze Zahl von 5 bis 150 ist; SS ein fester Träger ist; A eine geradkettige oder verzweigte C1- bis C10- Alkyl-Gruppe ist; und Rl, R2 und R3 unabhängig von einander ausgewählt sind aus der Gruppe bestehend aus Wasserstoff, Alkyl-Gruppen und Aryl-Gruppen.

2. Verfahren nach Anspruch 1, worin der feste Träger ausgewählt ist aus der Gruppe bestehend aus aminofunktionalen Membranen, porösem Glas. Siliciumdioxid, Polystyrolen, Polydimethylacrylamiden, Baumwolle und Papier.

3. Verfahren nach Anspruch 2, worin das Polystyrol ausgewählt ist aus der Gruppe bestehend aus Aminopolystyrol, Aminomethylpolystyrol, Aminoacylpolystyrol und p-Methylbenzhydrylaminpolystyrol.

## Revendications (Revendications pour l'(les) Etat(s) contractant(s) suivant(s): BE, CH, LI, DE, DK, FR, GB, IT, NL, SE)

1. Résine pour synthèse peptididique en phase solide représentée par la formule générale : 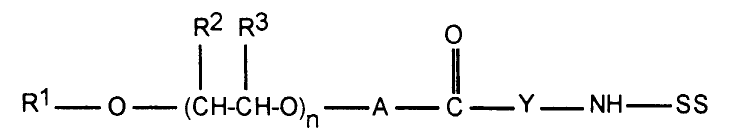 dans laquelle Y est un résidu d'acide diamino monocarboxylique qui peut être éventuellement protégé par un groupe protecteur N^{ω} ; n est un entier de 5 à 150 ; SS est un support solide ; A est un groupe alkyle en C₁-C₁₀ en forme de chaîne linéaire ou ramifiée ; et R¹, R² et R³ sont choisis indépendamment l'un de l'autre parmi le groupe consistant en l'atome d'hydrogène, les groupes alkyle et les groupes aryle.

2. Résine selon la revendication 1 dans laquelle le support solide est choisi parmi les membranes amino fonctionnelles, le verre poreux, la silice, les polystyrènes, les polydiméthylacrylamides, le coton et le papier.

3. Résine selon la revendication 2, dans laquelle le polystyrène est choisi parmi l'aminopolystyrène, l'aminométhyl-polystyrène, l'aminoacylpolystyrène et le p-méthylbenzhydrylamine-polystyrène.

## Revendications (Revendications pour l'(les) Etat(s) contractant(s) suivant(s): ES)

1. Procédé de fabrication d'un support pour synthèse pepdidique en phase solide comprenant la fabrication du support à partir d'une résine représentée par la formule générale : 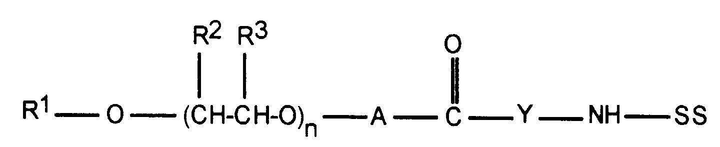 dans laquelle Y est un résidu d'acide diamino monocarboxylique qui peut être éventuellement protégé par un groupe protecteur N^{ω} ; n est un entier de 5 à 150 ; SS est un support solide ; A est un groupe alkyle en C₁-C₁₀ en forme de chaîne linéaire ou ramifiée ; et R¹, R² et R³ sont choisis indépendamment l'un de l'autre parmi le groupe consistant en l'atome d'hydrogène, les groupes alkyle et les groupes aryle.

2. Procédé selon la revendication 1 dans lequel le support solide est choisi parmi les membranes amino fonctionnelles, le verre poreux, la silice, les polystyrènes, les polydiméthylacrylamides, le coton et le papier.

3. Procédé selon la revendication 2, dans lequel le polystyrène est choisi parmi l'aminopolystyrène, l'aminométhyl-polystyrène, l'aminoacylpolystyrène et le p-méthylbenzhydrylamine-polystyrène.
